# EUROPEAN PATENT SPECIFICATION

(11) **EP 1 735 037 B1**
(45) Date of publication and mention of the grant of the patent: **20.05.2015**
(21) Application number: 05706274.7
(22) Date of filing: 23.02.2005
(51) Int. Cl.: A61M 25/00

(54) **ISOLATING CARDIAC CIRCULATION**
ISOLIERUNG DES HERZKREISLAUFS
ISOLEMENT DE CIRCULATION CARDIAQUE

(30) Priority: 26.02.2004 US 548038 P; 24.09.2004 US 612846 P
(43) Date of publication of application: 27.12.2006
(73) Proprietor: Osprey Medical Inc., Minnetonka, MN 55343 (US)
(72) Inventor: KAYE, David, Martin, Beaumaris, Victoria 3193 (AU); ALFERNESS, Clifton A, Washington 98367 (US); POWER, John, Melmouth, Williamstown, Victoria 3016 (AU); BILNEY, Adam, Lucas, Wy Yung, Victoria 3875 (AU)
(74) Representative: Di Gennaro, Sergio
(86) International application number: PCT/AU2005/000237
(87) International publication number: WO 2005/082440

(56) References cited:
- EP-A2- 0 301 854
- EP-B1- 0 150 960
- EP-B1- 0 526 102
- WO-A1-92/20387
- WO-A1-99/29227
- WO-A1-99/30765
- DE-A1- 10 102 045
- US-A- 4 795 427
- US-A1- 2002 091 349
- US-B1- 6 595 963
- US-B1- 6 673 039

## Description

### FIELD OF THE INVENTION

The present invention relates to the field of cardiology and more specifically to isolation of the cardiac circulation from the systemic circulation.

### BACKGROUND TO THE INVENTION

Heart disease is a major public health issue of very high prevalence, especially in the Western world. Cardiac conditions include coronary artery disease, ischaemic heart disease, heart failure, valvular heart disease, cardiac arrhythmias and cardiac inflammation (myocarditis) to name a few. Coronary artery disease and heart failure are possibly the most serious and prevalent, together being a leading cause of death in the Western world. The impact of acute myocardial infarction and congestive heart failure and their sequelae on the quality of life of patients and the cost of health care drives the search for new therapies.

While there is continual discovery of new and efficacious compounds to treat heart disease, delivery of the active agent to cardiac tissue can be problematic. For example, the structure of many pharmaceuticals may be altered by the liver, destroying their therapeutic activity. Accordingly, systemic administration (i.e. by oral, IV, IM routes and the like) is often sub optimal. This problem has been overcome in part by using sublingual or rectal administration to avoid "first pass" degradation through the liver. However, after these routes of administration the drug can still be degraded on subsequent passes through the liver.

Another problem relates to toxicity of therapeutic agents. For example, a drug administered to target a tumor of the heart may have a toxic effect on healthy tissue in other parts of the body. Indeed, anticancer treatments are often discontinued due to toxicity problems, frequently leading to further progression of the cancer.

Another problem in the delivery of therapeutic agents to tissues of the heart arises where agents intended for treatment of the heart alone are lost to the systemic circulation where they are metabolized without benefit, or have a deleterious effect on other healthy tissues. In some cases, significant amounts of the therapeutic agent may be needed, and efficiency of the treatment is therefore reduced by loss of the agent to the general circulation and time of exposure to the heart tissue.

In United States Patent Application 20020062121 (Tryggvason et al.), there is exemplified a method for the delivery of gene therapy pharmaceuticals to the liver and lung of a mammal utilizing a dosed perfusion system. While this document demonstrates some success in perfusing organs that have a comparatively simple vasculature, the document fails to disclose methods useful for delivering therapeutics to more complex organs.

EP-A-0301854 discloses a cardiovascular cannula for use particularly in situations requiring varying forms of cardiopulmonary bypass. The cannula which can be introduced percutaneously has an expandable section in proximity to drainage inlets. In one embodiment, the cannula is introduced percutaneously with the expansion means collapsed after the cannula has been introduced percutaneously through the vein into the right atrium the expansion means is employed to prevent the atrium from collapsing around and possibly impeding flow through the cannula, for instance during percutaneous initiation of cardiopulmonary bypass or other venous drainage application.

EP-B-0150960 discloses an inflatable catheter and method of medical treatment in which the flexible balloon material stretches when inflated to produce an acorn-shaped balloon, the broadened base of which seals against the vein interfacing the coronary sinus orifice of the heart to allow for stoppage of the natural flow of blood in the vessel, and which facilitates anterograde controlled, synchronous retroinfusion of pharmacologic or angiographic agent into the myocardium. This document further discloses an inflated catheter and method of medical treatment in which the apex of the inflated acorn-shaped balloon tapers away from the walls of the coronary sinus to prevent blockage of fluid flow through the middle cardiac vein thereby preventing pressure build up and edema in the myocardium.

US-B-6673039 discloses compositions, methods, kits, and apparatus which are provided for delivering a macromolecular assembly such as a plasmid, virus vector, or other gene vector, to an extravascular tissue such as muscle tissue. The composition comprises the macromolecular assembly and a vascular permeability-enhancing agent. In another embodiment, the composition further comprises a vasodilating agent. The disclosed method comprises proving a vascular permeability-enhancing agent to a blood vessel and providing a macromolecular assembly to the vessel. An oxygenator useful for providing oxygen to a fluid extracorporeally prior to providing the fluid to a blood vessel of a mammal is disclosed. Kits, apparatus, and methods for using the catheters disclosed therein for isolating cardiac circulation, diverting caval blood flow from the right atrium, and for other purposes, are also described.

US 2002/091349 A1 discloses a method and device for preventing contrast associated nephropathy. When contrast solution is injected into the coronary artery of a patient, blood is prevented from flowing through the coronary sinus into the right atrium. The blood in the coronary sinus is bypassed to a filtration device which filters out the contrast solution from the blood and recirculates the blood back to the patient. Preferably, blood flow from the coronary sinus is blocked by a balloon catheter which includes a port distal of the balloon so that when the sinus is occluded, blood flows from the sinus into the central lumen of the catheter where it can be directed to the filtration device. The preamble of claim 1 is based on this prior art.

It is an object of the present invention to overcome or alleviate a problem of the prior art by providing an improved method of isolating the cardiac circulation from the systemic circulation.

The discussion of documents, acts, materials, devices, articles and the like Is Included In this specification solely for the purpose of providing a context for the present invention. It is not suggested or represented that any or all of these matters formed part of the prior art base or were common general knowledge In the field relevant to the present invention as it existed before the priority date of each claim of this application.

### IN THE FIGURES

FIG 1 is a simplified illustration of human heart showing some of the cardiac veins and arteries which make up the coronary circulation.
FIG 2 is a flow diagram illustrating steps performed in a method of Isolating cardiac circulation according to an embodiment of the present intention.
FIG 3A illustrates a venous collection device having a support structure in the form of a frame.
FIG 3B illustrates an alternative venous collection device having a support structure which is a variation of the support structure illustrated In FIG 3A.
FIG 3C illustrates another venous collection device incorporating a woven support structure.
FIG 3D illustrates yet another venous collection device Incorporating a flange.
FIG 3E illustrates an embodiment of a venous collection device having a support structure which incorporates an inflatable balloon.
FIG 3F illustrates a cross section view of the support structure of FIG 3E.
FIG 3G illustrates a further embodiment of a venous collection device which Incorporates a support structure which is a variation of the support structure illustrated in FIG 3E.
FIG 4A illustrates a delivery device for use with an embodiment of the invention showing an un-inflated occluding means.
FIG 4B illustrates the delivery device of FIG 4A with the occluding means inflated.

### SUMMARY OF THE INVENTION

The present invention provides a percutaneously deliverable support structure for maintaining patency of the coronary sinus during collection of fluid therefrom, according to claim 1. The support structure comprises two consecutively inflatable regions. The first region is configured to, when inflated, rest in abutment with a portion of the right atrium wall surrounding the coronary sinus ostium. The second region is configured to, when inflated, maintain patency of the coronary sinus while occluding flow between the coronary sinus and the right atrium.

### DETAILED DESCRIPTION OF THE INVENTION

Delivering therapeutic agents to the heart for treatment of the heart tissue is more complicated than treatment of other organs because the heart must generate cardiac output as well as provide its own blood supply. Isolation of the heart's own blood supply from the systemic circulation is therefore desirable but challenging because of the potential variation between patients in cardiac vasculature, and the complex topography which has in the past deterred attempts at isolation.

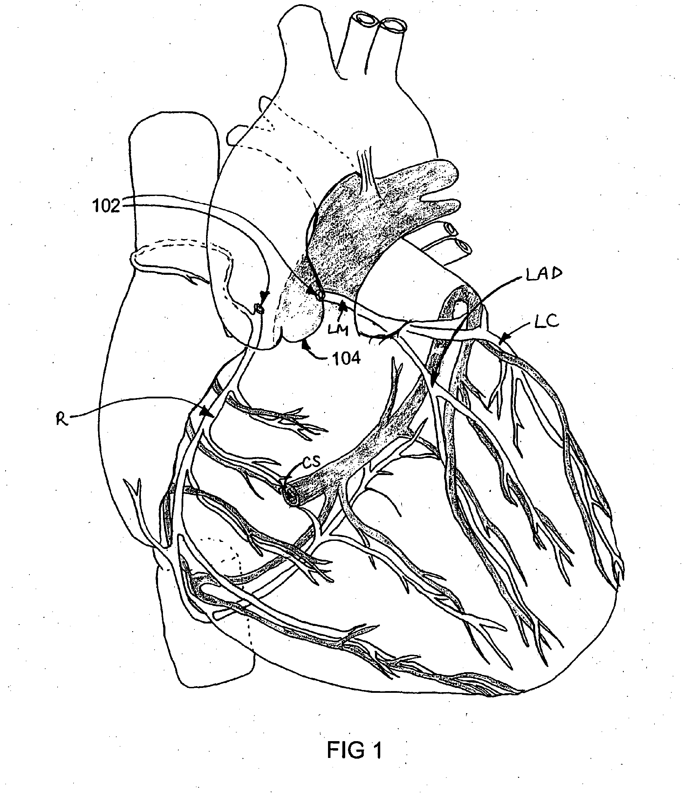

Normally, four main coronary arteries provide oxygenated blood to the heart for distribution throughout the heart tissue; the left interior descending (LAD), left circumflex (LC), left main (LM) and Right (R) coronary arteries. These are shown in the illustration of the heart provided in FIG 1. The coronary ostia 102 opening into these arteries are generally found in the aortic sinus, just above the cusps of the aortic valve 104, below the sinotubular junction. However, in a number of patients additional ostia are found in this region which open into accessory conal branches. These accessory ostia are usually smaller in diameter and irregularly located and are therefore difficult to isolate or catheterize using traditional approaches.

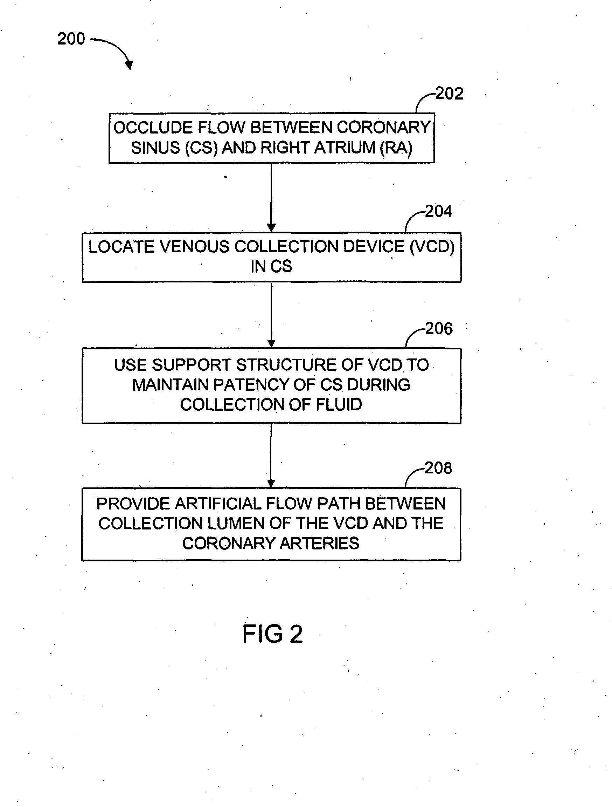

Referring now to FIG 2, a flow diagram shows steps involved in a method, generally referred to at 200, for substantially isolating cardiac circulation from systemic circulation. In a first step 202, flow between the coronary sinus (referred to as CS in FIG 1) and the right atrium is occluded. In a second step 204, a venous collection device having a collection lumen and a support structure is located in the coronary sinus. In a third step 206, the support structure is used to maintain patency of the coronary sinus during collection of fluid through the collection lumen. In another step 208, an artificial flow path is provided between the coronary sinus and the one or more coronary arteries. This method makes cardiac pumping for maintenance of systemic circulation achievable while the cardiac circulation is substantially isolated.

The steps of occluding flow between the coronary sinus and the right atrium and positioning the venous collection device in the coronary sinus may be performed in sequence or substantially simultaneously. Where these steps are performed substantially simultaneously, the venous collection device may also be an occluding device.

During isolation of the cardiac circulation from the systemic circulation using the method, it is important to maintain continuous circulation of fluid in the artificial flow path and in the cardiac circulation. Flow must be maintained to ensure delivery of blood (carrying oxygen and nutrients) to the cardiac tissue, and adequate delivery of therapeutic agents, if they are being administered.

In other organs, maintenance of desired flow rates during shunting of blood (e.g. in kidney dialysis) is relatively straightforward because the vessels through which access is gained are stiff and can withstand (a) insertion of collection catheters through the vessel wall, (b) occasional contact of the collection catheter tip with the vessel wall, and (c) negative pressures generated at the catheter tip during collection of fluid. In these cases, vessel collapse resulting from contact between the catheter tip and the vessel wall is rare, and not therefore of great concern during shunting procedures.

In contrast, the coronary sinus is significantly more difficult to deal with when attempting to collect fluid using a collection lumen located within the sinus. This is in part due to the fact that the coronary sinus wall is soft and conformable, unlike stiff artery walls, and is therefore prone to collapse when contacted by a collection catheter tip. This problem is intensified as a result of the negative pressures which may be generated at the catheter tip as fluid is drawn out of the sinus.

Further, because of the curvature of the coronary sinus, there is a natural tendency for a catheter tip approaching from the right atrium to contact the sinus wall, thus increasing the risk of collapse. Collapsing of the coronary sinus can cause venous pooling in the coronary veins and may therefore be fatal. Use of a venous collection device support structure to maintain patency of the coronary sinus, in accordance with the present invention, therefore minimizes the risk of these complications eventuating.

The venous collection device includes a collection lumen, an occluding body and a support structure which is configured to maintain patency of coronary sinus. The support structure also maintains the tip of the collection lumen substantially centrally, relative to the walls of the coronary sinus thereby further reducing the risk of the tip contacting the vessel wall. This is particularly important because, as briefly mentioned, a small negative pressure is established at the catheter tip as venous blood is drawn out of the sinus through the collection lumen. This negative pressure increases the risk of damaging the wall of the coronary sinus if contacted by the tip and also increases the risk of the collection lumen being occluded (i.e. by the vessel wall). Centralizing the collection lumen using the support structure significantly reduces the risk of invagination of the tip of collection lumen into the coronary sinus wall.

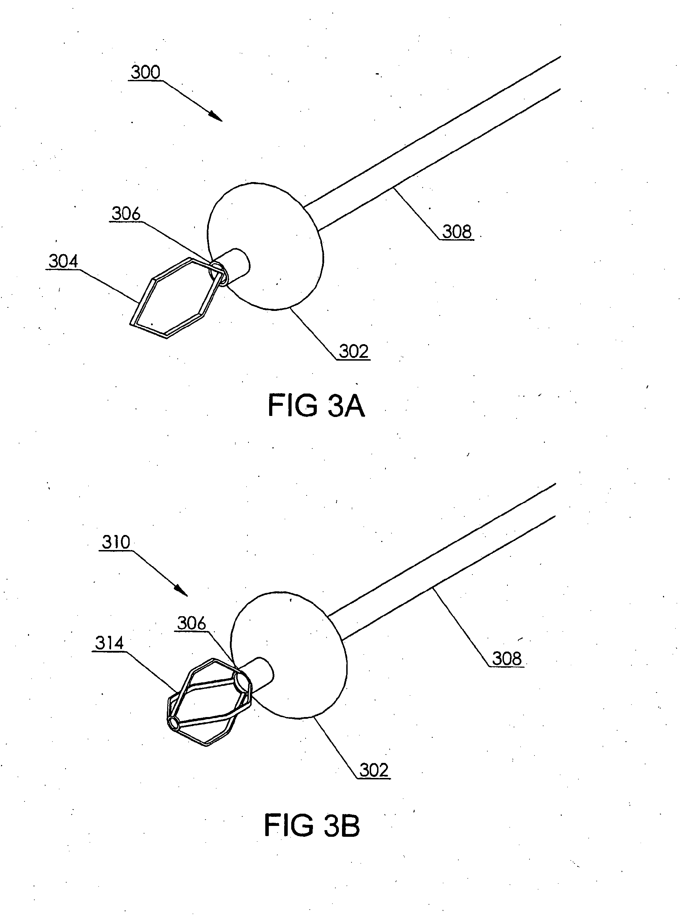

The venous collection device may be embodied in many different forms. FIGs 3A to 3G illustrate some examples of different embodiments 300, 310 320, 330, 340 and 350 of a venous collection device, of which 340 and 350 are embodiments according to the invention. Preferably, the tip of the venous collection device is positioned just inside the coronary sinus ostium to ensure that venous blood is collected from all or at least most of the coronary veins draining into the coronary sinus.

FIG 3A shows a venous collection device 300 having an occluding portion in the form of inflatable balloon 302 and a support structure in the form of a two-dimensional frame 304. Frame 304 is designed to compress or fold down so that it can be delivered percutaneously, and expand when released in position in the coronary sinus. When expanded, the frame contacts opposing walls of the coronary sinus, thereby centralizing the tip 306 of the collection lumen 308 within the sinus. Venous blood is then collected from the sinus through collection lumen 308 and channeled into the artificial flow path.

The venous collection device 310 of FIG 3B substantially replicates the support structure illustrated in FIG 3A with the exception that the support structure of FIG 3B is a three-dimensional frame 314 which opens to support the vessel wall in width and in height. Frame 314 is also designed to compress or fold down for percutaneous delivery. It is to be understood that expansion of the folded frame may be provided in a number of ways. In the embodiment illustrated, frame 314 is provided by two substantially hexagonal frame pieces arranged at right angles in such a way that they are compressible or collapsible for percutaneous delivery, and then open out to a "supporting" configuration when positioned within the coronary sinus. Other support structures may be sprung, hinged or use other suitable compression, folding and expansion mechanisms to achieve the desired functionality. Frame segments may incorporate a range of different geometrical configurations, including oblong, oval and the like, if a frame arrangement is adopted

In the embodiments illustrated in FIG 3A and FIG 3B, support structure 304 or 314 may be manipulated using a guide wire passing through collection lumen 308. To deploy venous collection device 300 or 310, collection lumen 308 is delivered percutaneously and tip 306 is positioned just inside the coronary sinus. When tip 306 is in place (as determined by any suitable imaging technique), balloon 302 is inflated to occlude flow between the coronary sinus and right atrium. Support structure 304 or 314 is compressed or folded so as to fit inside collection lumen 308. The compressed support structure is delivered, inside collection lumen 308 and through the opening at tip 306 to the coronary sinus. As support structure 304 or 314 is pushed out of the collection lumen through the opening at tip 306, it expands to its "supporting configuration", thereby maintaining patency of the vessel and centralizing tip 306 of collection lumen 308 relative to the sinus walls.

It is to be understood that the support structure may be attached to or delivered within the collection lumen (as described above), or it may be provided as a separate component deliverable through a delivery catheter or sheath which may also deliver or position the collection lumen and/or occluding balloon. In such an embodiment, once the support structure is positioned in the coronary sinus, the delivery catheter/sheath is retracted relative to the support structure enabling it to expand and support the sinus walls to maintain patency. Centralization of the collection lumen tip is also achieved. The delivery catheter is then removed from the patient.

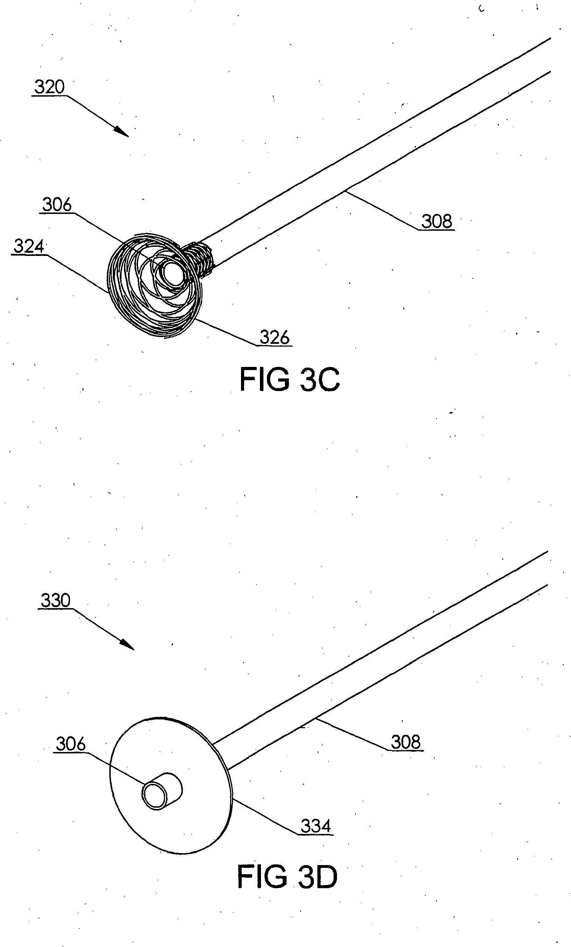

The venous collection device 320 illustrated in FIG 3C takes a different form. In this embodiment, the support structure 324 is an expandable framework having a woven or braided, basket-like configuration when expanded. In place of the inflatable occluding balloon 302, support structure 324 also has a thin silicon or other flow-proof coating 326 on the inner and/or outer surface of the framework to prevent flow between the coronary sinus and the right atrium. In a manner similar to the embodiments illustrated in FIGs 3A and 3B, the embodiment illustrated in FIG 3C maintains patency of the coronary sinus and centralizes the tip 306 of collection lumen 308.

The support structure 324 can be compressed or minimized for percutaneous delivery to the coronary sinus via a delivery catheter (not shown), as is the case for the devices illustrated in FIGs 3A and 3B. This may be achieved by elongating the braid so as to reduce the diameter of the device. When in position, the delivery catheter is retracted and the support structure 324 expands to contact the inner wall of the coronary sinus, holding the sinus open and centralizing the tip 306 of the collection lumen 308 with respect to the vessel wall.

Guide wires or other ancillary fibers/devices may aid in the positioning and expansion of the support structure. When the support structure is expanded, the silicon sheath or other flow-proof coating becomes taught, occluding flow between the sinus and the right atrium. Preferably, the rim of support structure 324 has a soft coating to minimize damage to the sinus wall. It is to be understood that the support structure 324 of FIG 3C is only one example embodiment and that other forms may be adopted, such as telescopically and radially expandable frameworks.

Preferably, the support structures 304, 314 and 324 of FIGs 3A, 3B and 3C respectively are formed from a biocompatible shape memory material. Such materials include nitinol, a shape memory alloy. Nitinol devices can be manufactured in such a way that they can be compressed for percutaneous delivery to a deployment site and then resume a "shape memory" configuration when released from the delivery lumen into the temperate environment of the blood vessel. It is to be understood, however, that other biocompatible materials including plastics (e.g. hydrophilic plastics), ceramics and the like may also be suitable.

To minimize the risk of blocking small veins which feed into the coronary sinus, the venous collection device should sit just inside the coronary sinus ostium, or be pressed against the ostium from the right atrium chamber. In either case, a hemodynamic seal is established. It is also desirable for the collection lumen 308 to be flexible for ease of delivery and positioning within the coronary sinus and to prevent vessel tenting.

The venous collection device 330 illustrated in FIG 3D takes yet another form. Here, occlusion is achieved by positioning flange 334 over the coronary sinus ostium, closing it from the atrial side. In this embodiment, the venous collection device should be selected with a flange diameter which is sufficient to block flow through the coronary sinus ostium when it is in abutment with the surrounding portion of the right atrium wall. Tip 306 of the collection lumen 308 sits just inside the coronary sinus, protruding centrally of the flange 334. Advantageously, the negative pressure generated within the sinus during collection of venous flow creates a more effective seal between the flange 334 and the coronary sinus ostia, preventing flow from the isolated cardiac circulation into the systemic circulation via the right atrium. To achieve improved patency, the flange configuration of FIG 3D may be combined with either of the support structures illustrated in FIGs 3A and 3B.

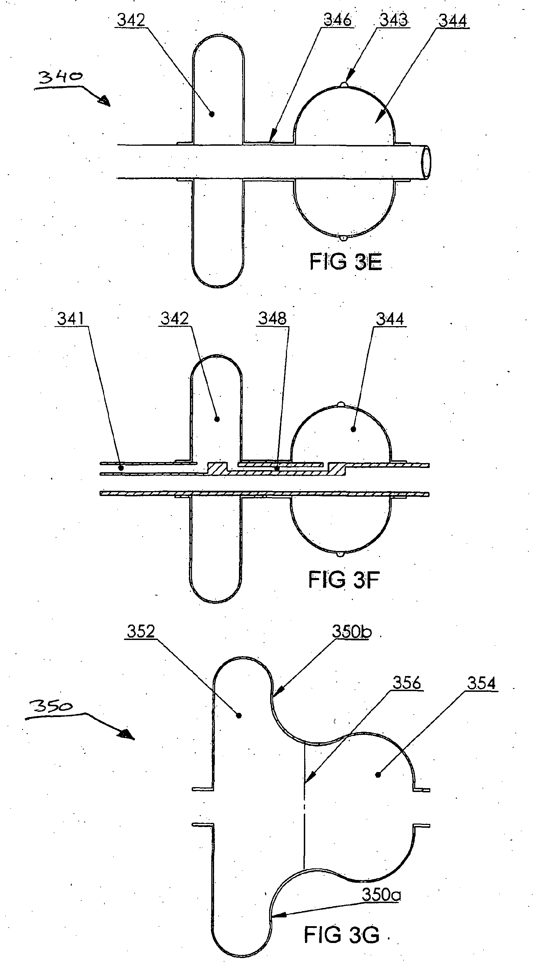

FIGs 3E and 3G illustrate venous collection devices 340, 350, each having a slightly different support structure which incorporates two consecutively inflatable regions. The first region is flange-like in shape and is configured to, when inflated, rest in abutment with a portion of the right atrium wall surrounding the coronary sinus ostium. When inflated, the second region sits inside the coronary sinus, contacting the vessel wall to form a seal and occlude flow between the sinus and the right atrium, whilst maintaining patency of the sinus while fluid is collected by the collection lumen. Collection lumen 308 extends through the balloon arrangement providing the added advantage of centralizing the tip 306 with respect to the sinus, further reducing the risk of invagination into the vessel wall and collapsing of the sinus.

To deploy such a device, a guide wire (not shown) placed inside the coronary sinus guides the inflatable support structure into position. The first (proximal) region is inflated and a collection catheter extending therethrough is moved toward the coronary sinus ostium. Location of the ostium can be determined by deformation of the first (proximal) region. When the proximal region is inflated with a fluid which includes a contrast solution, radiographic imaging may be used. When in position, the second (distal) region is inflated to occlude flow between the sinus and the right atrium, maintaining patency of the sinus and centralizing the catheter tip for collection of fluid. This may be achieved by inflating two separate balloons or two conjoined inflatable balloon regions. The former requires incorporation of 3 lumens to enable (i) collection of fluid from the sinus; (ii) inflation of the first (proximal) balloon; and (iii) inflation of the second (distal) balloon whereas the latter requires only 2 lumens

In the embodiments illustrated in FIGs 3E and 3G, the first and second inflatable regions are distinguished by a pressure-sensitive actuator which facilitates consecutive inflation of the first and second regions. When the first region has been inflated and a pre-determined pressure differential is established across the actuator, inflation of the second region occurs. The actuator may be in the form of a valve, membrane or conduit system. Advantageously, in the embodiments illustrated in FIGs 3E and 3G, the first inflating region acts like an anchor, precisely locating the inflation site of the second inflating region.

Referring now to the particular embodiment illustrated in FIG 3E, the first region is provided in the form of a first inflatable body 342 and the second region is provided in the form of a second inflatable body 344. The two regions are connected by a neck 346. In such an arrangement, the distance between the first and second inflatable bodies is pre-determined by the length of the neck, and this can be used to position the second inflatable body in the sinus accurately, prior to inflation. When the neck length is selected appropriately, occlusion of flow between the coronary sinus and the right atrium can be achieved whilst permitting collection of blood from substantially all of the tributary coronary veins feeding into the sinus, including the middle cardiac vein.

FIG 3F is a cross sectional view illustrating one arrangement for achieving consecutive inflation of the first and second inflatable bodies for the support structure illustrated in FIG 3E. In such an arrangement, neck 346 connecting first and second inflatable bodies 342, 344 includes a secondary inflation conduit 348 which is much narrower than main inflation conduit 341. When the first inflatable body is inflated to a certain pressure, fluid is then forced to escape the first body through the secondary inflation conduit enabling second inflation body to inflate. It is to be understood that other methods may be relied on to achieve consecutive inflation of the inflatable bodies. Such alternative methods may involve use of fluids having different viscosities (e.g. a contrast solution and saline) to inflate regions separately. The viscosity differential may be relied on alone, or in combination with other apparatus such as valves, capillaries and membranes.

The alternative support structure illustrated in FIG 3G is in the form of a bell with the first and second inflatable bodies, 352, 354 distinguished by a membrane 356 inside the structure. The flange portion (base) of the bell which houses the first inflatable body 352 is configured to sit with walls 350a in abutment with a portion of the right atrium wall surrounding the coronary sinus ostium. The second inflatable body 354 is provided in the dome of the bell. When a certain pressure develops inside first inflatable body 352, membrane 356 fails enabling flow of fluid into and inflation of second inflatable body 354.

Alternatively, a valve or other suitable actuator may be used in place of membrane 356. Such valves may permit bi-directional control of flow between the first and second inflating bodies facilitating easy removal of the structure at the end of a procedure. In another embodiment, the dome of the bell may be folded upon itself onto the flange portion for percutaneous delivery to the coronary sinus. In such arrangement, a membrane or valve may not be necessary as adhesion between the folded layers may be sufficient to facilitate differential (consecutive) inflation of the two parts. The second inflating region may also include securing ribs, abrasions, spikes or the like, 343 to enhance stability of the support structure inside the sinus.

In dual balloon arrangement of the embodiments illustrated in FIGs 3E and 3G, the first region has the capacity to provide a second level of occlusion, in addition the occlusion provided by the region of the balloon which is inflated inside the sinus. Advantageously, the seal may be improved as a result of the negative pressure generated within the sinus during collection of fluid. Guide wires and/or any other suitable ancillary devices may be used to deploy the inflatable support structure.

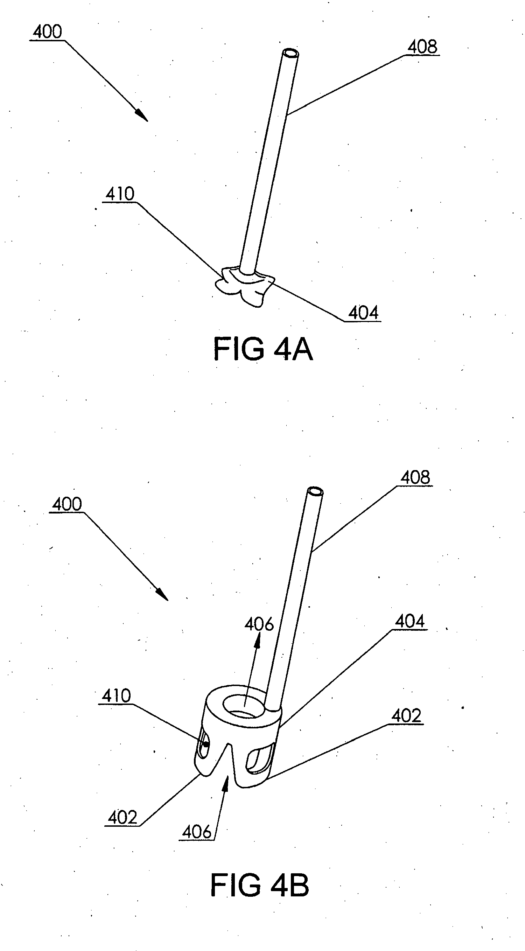

At the other end of the artificial flow path, a delivery device is positioned proximal the aortic valve to deliver blood (and agents) from the artificial flow path to the coronary arteries for circulation through the cardiac tissue. FIGs 4A and 4B illustrate an example of a delivery device for delivering flow from the artificial flow path to the coronary arteries. The illustrated device occludes flow between the aorta and the coronary arteries thereby completely isolating the cardiac circulation from the systemic circulation. However, it is to be understood that the delivery device may deliver flow from the artificial flow path with or without occluding flow of fresh systemic blood from the aorta into the coronary arteries. In some embodiments where fluid from the artificial flow path is delivered to the coronary arteries at a relatively low flow rate, it may be desirable to permit extra blood flow from the aorta into the coronary arteries to supplement flow to the cardiac tissue. This results in dilution of any therapeutic agent introduced via the artificial flow path. However, such dilution can be compensated by replenishing supply of the agent in the artificial flow path.

In other embodiments, dilution of therapeutic agent may be undesirable so flow from the aorta into the coronary arteries should be prevented or at least minimized. Accordingly, it may be desirable for the delivery device to occlude flow between the aorta and the coronary arteries. In a similar manner to the positioning of the venous collection device in the coronary sinus, the steps of positioning the delivery device and occluding flow between the aorta and the one or more coronary arteries may be performed in two separate steps or substantially simultaneously. Where these steps are performed substantially simultaneously, the delivery device may also be an occluding device. To achieve isolation of the artificial flow path supplying the coronary arteries from the systemic circulation, an occluding catheter may be used.

An occluding catheter for occluding flow between a main vessel and one or more branched vessels has a supply lumen and an inflatable body portion fed by the supply lumen. When inflated, the inflatable body portion occludes flow between the main vessel (aorta) and the one or more branched vessels (coronary arteries). The inflatable body portion has an opening which, when the body portion is inflated, creates one or more first flow channels between the supply lumen and one or more branched vessels (coronary arteries). When inflated, the inflatable body portion also forms a second flow channel which permits flow in the main vessel (aorta) across the inflatable body portion in isolation from the one or more first flow channels.

FIGs 4A and 4B show a delivery and occluding device 400. An inflatable annulus 404 is shown in an un-inflated state in FIG 4A and in an inflated state in FIG 4B. Inflatable annulus 404 is fed with fluid from the artificial flow path by delivery lumen 408 thereby causing it to inflate. Delivery lumen 408 is in turn fed by the artificial flow path (not shown). Inflatable annulus 404 includes an opening 410 configured to remain substantially closed when the delivery device is deployed to the aortic valve region, and to open when the annulus in position and inflated.

Alternatively, the opening may extend around a substantial portion of the circumference of annulus 404 providing a flow path between the delivery lumen 408 and coronary arteries extending from the aortic sinus. In such an arrangement, the opening may therefore also supply accessory conal branches which exist in some patients ensuring more complete treatment of the cardiac tissue by any therapeutic agent which is introduced via the artificial flow path. The delivery device may provide two (as illustrated) or more openings 410 positioned around annulus 404 in such a way that each opening is used to establish a flow path to the left main (LM) and right (R) coronary arteries separately and to accessory branches if present via additional openings (not shown). When inflated, delivery device 400 also provides a systemic flow channel 406 through the center of the annulus 404. Systemic flow channel 406 enables the heart to generate and maintain cardiac output to the rest of the body while the cardiac circulation is isolated.

In use, the delivery device 400 is delivered percutaneously, in a deflated state (FIG 4A), to the aorta and deployed just inside the cusp of the aortic valve. When in position, the annulus 404 is slowly inflated using supply from the artificial flow path, through delivery lumen 408. During delivery and deployment of the venous collection and delivery devices, it is important that their location and orientation is monitored. This is particularly important when deploying the delivery device, to ensure that openings 410 are positioned around the coronary ostia to enable flow between the artificial flow path and the coronary arteries. If the annulus is positioned in such a way that one or more of the coronary ostia is covered, inflation of the annulus is likely to occlude flow to the coronary arteries and cause serious damage to the cardiac tissue. Imaging techniques known in the art may be used, as well as guide wires, to aid in positioning the device.

It is desirable that the delivery device is sized appropriately to ensure a snug fit inside the aorta. Such snug fit minimizes leakage from the delivery lumen 408 into the aorta and substantially prevents flow from the aorta into the coronary arteries. In some cases this fit may be problematic because it impedes closure of the aortic valve leaflets. To address this issue, it is preferable that inflatable annulus 404 is contoured to prevent obstruction of valve closure. In such an embodiment, the delivery device further includes lobes 402 which correspond to each of the lobes of the aortic valve. Inclusion of the lobes enables the delivery device to be positioned snuggly inside the aortic valve whilst permitting valve closure.

A delivery device of the kind illustrated in FIGs 4A and 4B is suitable for delivering a blood (and therapeutic agent) solution from the artificial flow path to the coronary arteries without delivering the solution to the systemic circulation. The blood/therapeutic agent solution may then be collected after passing through the heart tissue using a venous collection device, as described previously. Collected solution can then be re-circulated through the heart with re-oxygenated collected blood. Such perfusion method is beneficial as it maximizes the efficiency of the therapeutic agent by eliminating break down in the liver and stomach, and also reduces or eliminates the toxicity issues associated with specific treatments reaching non-target tissue. Re-circulation further improves the uptake of therapeutic agents by increasing the exposure time to the target tissue, improving the effectiveness of treatment and reducing the cost of treatment by limiting uncontrolled loss of therapeutic agent to the systemic circulation.

As an alternative to the delivery device of FIGs 4A and 4B, coronary artery occluding catheters may be used to supply the coronary arteries with the blood solution from the artificial flow path, and occlude flow from the aorta into the coronary arteries. Alternatively, catheters which do not occlude flow from the aorta into the coronary arteries may be used to supply flow from the artificial flow path into the coronary arteries. However, this will result in dilution of any therapeutic agent in the solution being circulated.

Venous blood in the circulating solution will become oxygen depleted after supplying oxygen to the cardiac tissue. Therefore, it is desirable to include in the artificial flow path an oxygenation system, preferably of the kind normally incorporated into a cardiopulmonary bypass system or extracorporeal membrane oxygenation (ECMO) or equivalent. Using such a system, venous blood collected from the coronary sinus is oxygenated in the artificial flow path prior to it being re-circulated back into the heart. Therapeutic agent in the blood can also be replenished before re-circulation.

Means for circulating the blood solution in the artificial flow path (and through the cardiac tissue) may be provided in a range of different forms as would be appreciated by the skilled addressee. Such means may comprise a pulsatile or rotary pump incorporated into the apparatus to generate the required pressure head to circulate the blood. Such pressure is desired to be in the range 50 to 80mmHg. To perfuse the blood/agent solution into the coronary arteries, the blood/agent solution should be drawn from the venous collection device at a suitable rate. It has been found that a rate of approximately 180 to 200 milliliters per minute may be suitable for most adults although flow rates as high as 250 milliliters per minute may be required. It is to be understood that this rate is not limiting (nor is the suggested pressure head), and may be adjusted according to the size, age and condition of the patient, and the nature of the apparatus and components used.

An auxiliary flow channel connected to a back-up reservoir may also be provided in the artificial flow path, to provide the requisite pressure head if constant flow at the desired rate cannot be achieved naturally. Alternatively, the auxiliary flow channel may draw blood from the right atrium to supplement the flow rate from the coronary sinus. Preferably, the artificial flow path includes means to monitor and adjust flow rates to ensure adequate supply to the coronary arteries. For example, where flow rates measured at the coronary sinus indicate that there is insufficient blood (and therapeutic agent) supply to the coronary arteries, a slow release valve may be activated which results in increased blood flow. This may also be in response to a negative pressure detected at the pump. The auxiliary flow channel enables more blood to enter the cardiac circulation with out compromising isolation of the therapeutic agent or other perfusate. The collection lumen and tip thereof should be sufficiently large to support the required flow rate.

Therapeutic agents and substances which may be added to the solution in the flow path may be selected from the group consisting of one or more of a virus, a pharmaceutical, a peptide, a hormone, a stem cell, a cytokine, an enzyme, a gene therapy agent, blood and blood serum. Advantageously, the present invention enables treatment of the heart tissue by gene therapy or the like, in the beating heart Such treatment has not hitherto been achievable.

It is to be understood that while embodiments of the present invention have been described in the context of anterograde circulation and perfusion, it is to be understood that aspects of the invention may also be suitable for retrograde perfusion of the cardiac tissue and calls thereof.

## Claims

1. A system comprising a percutaneously deliverable apparatus support structure for maintaining patency of the coronary sinus during collection of fluid therefrom, the percutaneously deliverable apparatus support structure comprising two consecutively inflatable regions wherein:
a collection lumen (308) comprising a centralized tip (306);
a first inflatable region (342, 352), wherein the collection lumen extends through the first inflatable region, wherein the first inflatable region is configured to, when inflated, rest in abutment with a portion of the right atrium wall surrounding the coronary sinus ostium; and
a second inflatable region (344, 354), **characterised in that**:
the collection lumen extends through the second inflatable region, wherein the second inflatable region is configured to, when inflated, sit inside the coronary sinus and occlude flow between the coronary sinus and the right atrium, whilst allowing collection of fluid from the coronary sinus through a the centralized tip of the collection lumen.

2. A system percutaneously deliverable support structure according to claim 1, wherein the first inflatable region and the second region are distinguished by a pressure- sensitive actuator which facilitates inflation of the first inflatable region substantially independently of the second inflatable region, until a pre-determined pressure differential is established across the actuator which facilitates inflation of the second inflatable region.

3. A system percutaneously deliverable support structure according to claim 1, wherein the first region is provided by a first inflatable body and the second region is provided by a second inflatable body and the first and second inflatable regions bodies are connected by a channel which facilitates fluid flow from the first inflatable region into the second inflatable region for inflation thereof, after inflation of the first inflatable region.

4. A system percutaneously deliverable support structure according to claim 3, wherein the channel has a length which is pre-determined to facilitate accurate positioning of the second inflatable region inside the coronary sinus.

5. A system percutaneously deliverable support structure of claim 1, wherein said support structure is a component of a cardiac circulation treatment system further comprising a delivery device for delivering substance to the one or more coronary arteries.

6. A system percutaneously deliverable support structure of claim 1, wherein said support structure is a component of a cardiac circulation treatment system further comprising a delivery device for delivering substance to the one or more coronary arteries, said delivery device further comprising occluding means configured to occlude blood flow between the aorta and one or more arteries.

7. A system percutaneously deliverable support structure of claim 1, wherein said support structure is a component of a cardiac circulation treatment system further comprising a delivery device for delivering substance to the one or more coronary arteries, said substance being a therapeutic substance selected from the group consisting of one or more of a virus, a pharmaceutical, a peptide, a hormone, a stem cell, a cytokine, an enzyme, a gene therapy agent, blood and blood serum.

## Patentansprüche

1. System mit einer Halterungsvorrichtung für eine perkutan zuführbare Einrichtung zur Aufrechterhaltung der Durchlässigkeit des Koronarsinus, während diesem Flüssigkeit entnommen wird, wobei die Halterungsvorrichtung zwei, aufeinanderfolgend aufblasbare Bereiche enthält mit:
einem Aufsammel-Lumen (308) mit einer zentriert gehaltenen Spitze (306),
einem ersten aufblasbaren Bereich (342, 352), durch den sich das AufsammelLumen erstreckt und der so ausgebildet ist, dass er im aufgeblasenen Zustand an einem Teil der rechten Vorhofwandung anliegt, welche die Koronarsinusöffnung umgibt, und
einem zweiten aufblasbaren Bereich (344, 354),
**dadurch gekennzeichnet, dass**
das Aufsammel-Lumen durch den zweiten aufblasbaren Bereich verläuft, welcher so ausgebildet ist, dass er im aufgeblasenen Zustand innerhalb des Koronarsinus sitzt und einen Fluss zwischen diesem und dem rechten Vorhof blockiert, jedoch ein Aufsammeln von Flüssigkeit von dem Koronarsinus durch die zentrierte Spitze des Aufsammel-Lumens erlaubt.

2. System nach Anspruch 1, bei welchem der erste aufblasbare Bereich und der zweite Bereich durch einen druckempfindlichen Aktuator getrennt sind, welcher ein Aufblasen des ersten aufblasbaren Bereichs im Wesentlichen unabhängig vom zweiten aufblasbaren Bereich erlaubt, bis sich ein vorbestimmter Druckunterschied über dem Aktuator aufbaut, welcher ein Aufblasen des zweiten aufblasbaren Bereichs ermöglicht.

3. System nach Anspruch 1, bei welchem der erste Bereich durch einen ersten aufblasbaren Körper und der zweite Bereich durch einen zweiten aufblasbaren Körper gebildet wird und der erste und der zweite aufblasbare Bereich durch einen Kanal miteinander verbunden sind, welcher einen Fluidfluss vom ersten aufblasbaren Bereich in den zweiten aufblasbaren Bereich erlaubt, um Letzteren nach dem Aufblasen des ersten aufblasbaren Bereichs aufzublasen.

4. System nach Anspruch 3, bei welchem die Länge des Kanals so bemessen ist, dass der zweite aufblasbare Bereich innerhalb des Koronarsinus genau positioniert ist.

5. System nach Anspruch 1, bei welchem die Halterungsvorrichtung Teil eines Herzkreislaufbehandlungssystems ist und welches weiterhin eine Zuführungseinrichtung zum Zuführen einer Substanz zu einer oder mehreren Koronararterien enthält.

6. System nach Anspruch 1, bei welchem die Halterungsvorrichtung Teil eines Herzkreislaufbehandlungssystems ist und welches ferner eine Zuführungseinrichtung zum Zuführen einer Substanz zu einer oder mehreren Koronararterien enthält und die Zuführungseinrichtung weiterhin eine so ausgebildete Verschlussvorrichtung enthält, dass ein Blutfluss zwischen der Aorta und einer oder mehreren Arterien blockiert wird.

7. System nach Anspruch 1, bei welchem die Halterungsvorrichtung Teil eines Herzkreislaufbehandlungssystems ist und welches ferner eine Zuführungseinrichtung zum Zuführen einer Substanz zu einer oder mehreren Koronararterien enthält, wobei diese Substanz eine therapeutische Substanz aus der Gruppe aus einer oder mehrerer der folgenden Substanzen ist: ein Virus, ein Pharmazeutikum, ein Peptid, ein Hormon, eine Stammzelle, ein Zytokin, ein Enzym, ein Gentherapiemittel, Blut oder Blutserum.

## Revendications

1. Système comprenant une structure de support d'appareil pouvant être administrée par voie percutanée pour maintenir la perméabilité du sinus coronaire pendant la collecte du fluide de ce dernier, la structure de support d'appareil pouvant être administrée par voie percutanée comprenant deux régions consécutivement gonflables, dans lequel :
une lumière de collecte (308) comprenant une pointe centralisée (306) ;
une première région gonflable (342, 352), dans lequel la lumière de collecte s'étend à travers la première région gonflable, dans lequel la première région gonflable est configurée, lorsqu'elle est gonflée, pour s'appuyer en butée contre une partie de la paroi de l'oreillette droite entourant l'ostium du sinus coronaire ; et
une seconde région gonflable (344, 354), **caractérisé en ce que** :
la lumière de collecte s'étend à travers la seconde région gonflable, dans lequel la seconde région gonflable est configurée, lorsqu'elle est gonflée, pour s'installer à l'intérieur du sinus coronaire et boucher l'écoulement entre le sinus coronaire et l'oreillette droite, tout en permettant la collecte du fluide du sinus coronaire par la pointe centralisée de la lumière de collecte.

2. Structure de support de système pouvant être administrée par voie percutanée selon la revendication 1, dans laquelle la première région gonflable et la seconde région sont distinguées par un actionneur sensible à la pression qui facilite le gonflage de la première région gonflable sensiblement indépendamment de la seconde région gonflable, jusqu'à ce qu'un différentiel de pression prédéterminé soit établi sur l'actionneur qui facilite le gonflage de la seconde région gonflable.

3. Structure de support de système pouvant être administrée par voie percutanée selon la revendication 1, dans laquelle la première région est fournie par un premier corps gonflable et la seconde région est fournie par un second corps gonflable et les premier et second corps de régions gonflables sont raccordés par un canal qui facilite l'écoulement de fluide à partir de la première région gonflable dans la seconde région gonflable pour son gonflage, après le gonflage de la première région gonflable.

4. Structure de support de système pouvant être administrée par voie percutanée selon la revendication 3, dans laquelle le canal a une longueur qui est prédéterminée pour faciliter le positionnement précis de la seconde région gonflable à l'intérieur du sinus coronaire.

5. Structure de support de système pouvant être administrée par voie percutanée selon la revendication 1, dans laquelle ladite structure de support est un composant d'un système de traitement de circulation cardiaque comprenant en outre un dispositif d'administration pour administrer une substance aux une ou plusieurs artères coronaires.

6. Structure de support de système pouvant être administrée par voie percutanée selon la revendication 1, dans laquelle ladite structure de support est un composant d'un système de traitement de circulation cardiaque comprenant en outre un dispositif d'administration pour administrer une substance aux une ou plusieurs artères coronaires, ledit dispositif d'administration comprenant en outre des moyens d'occlusion configurés pour boucher l'écoulement de sang entre l'aorte et les une ou plusieurs artères.

7. Structure de support de système pouvant être administrée par voie percutanée selon la revendication 1, dans laquelle ladite structure de support est un composant d'un système de traitement de circulation cardiaque comprenant en outre un dispositif d'administration pour administrer la substance aux une ou plusieurs artères coronaires, ladite substance étant une substance thérapeutique choisie dans le groupe comprend un ou plusieurs parmi un virus, un produit pharmaceutique, un peptide, une hormone, une cellule souche, une cytokine, une enzyme, un agent de thérapie génique, du sang et un sérum sanguin.
